(19) Europäisches Patentamt
European Patent Office
Office européen des brevets

(11) Veröffentlichungsnummer: **0 523 545 B1**

# (12) EUROPÄISCHE PATENTSCHRIFT

(45) Veröffentlichungstag der Patentschrift: **30.08.95**

(51) Int. Cl.$^6$: **A61K 6/093**, A61K 6/083

(21) Anmeldenummer: **92111661.2**

(22) Anmeldetag: **09.07.92**

Die Akte enthält technische Angaben, die nach dem Eingang der Anmeldung eingereicht wurden und die nicht in dieser Patentschrift enthalten sind.

## (54) Dentalmaterial mit Alumoorganopolysiloxan-Füllstoff.

(30) Priorität: **19.07.91 DE 4123946**

(43) Veröffentlichungstag der Anmeldung:
**20.01.93 Patentblatt 93/03**

(45) Bekanntmachung des Hinweises auf die Patenterteilung:
**30.08.95 Patentblatt 95/35**

(84) Benannte Vertragsstaaten:
**AT BE CH DE DK ES FR GB IT LI LU MC NL PT SE**

(56) Entgegenhaltungen:
**EP-A- 0 358 011**
**EP-A- 0 394 797**
**EP-A- 0 394 798**
**EP-A- 0 399 148**
**WO-A-92/16183**

(73) Patentinhaber: **Degussa Aktiengesellschaft**
**Weissfrauenstrasse 9**
**D-60311 Frankfurt (DE)**

(72) Erfinder: **Panster, Peter, Dr.**
**Im Lochseif 8**
**W-6458 Rodenbach (DE)**
Erfinder: **Jänes, Arno**
**Am Steinacker 13**
**W-6467 Hasselroth 2 (DE)**
Erfinder: **Mackert, Wilhelm**
**Nordring 37**
**W-6458 Rodenbach (DE)**
Erfinder: **Stange, Michael**
**Kasselbergweg 38**
**W-6482 Bad Orb (DE)**
Erfinder: **Rentsch, Harald, Dr.**
**Grünaustrasse 17**
**W-6450 Hanau 9 (DE)**

EP 0 523 545 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein pastöses, in Gegenwart eines Initiators zu einer auf Hochglanz polierbaren Masse aushärtbares Dentalmaterial aus einem polymerisierbaren organischen Bindemittel und einem feinteiligen Füllstoff. Das Material enthält als Bindemittel mindestens ein polymerisierbares Methacrylat und einen gegebenenfalls silanisierbaren neuartigen Füllstoff auf Basis Aluminium-haltiger Polysiloxane. Daneben können Initiatoren zur Polymerisationsauslösung, weitere Füllstoffe wie fein gemahlene Gläser, hochdisperse Kieselsäure oder vorgebildete Polymerisate, Pigmente und Stabilisatoren enthalten sein. Auch andere Additive wie Weichmacher oder Schlagzähigkeitsverbesserer kommen in Frage.

Der Begriff "Dentalmaterial" umfaßt beispielsweise Füllungsmaterialien, um kariöse Defekte oder andere Zahndefekte im Mundraum zu versorgen, Inlays, Kronen-und Brückenmaterialien, Verblendungen, Versiegelungs- und Schutzüberzugsmassen, Kunststoffbefestigungsmaterialien zum Festsetzen von Inlays oder Kronen und Brücken, Stumpfaufbaumaterialien, Prothesenmaterialien sowie Massen zur Herstellung von künstlichen Zähnen.

Übliche Dentalmassen der obengenannten Art enthalten mindestens einen monomeren Ester der Methacrylsäure, meist aber ein Gemisch aus mehreren solcher Ester. Geeignete monofunktionelle Ester der Methacrylsäure sind beispielsweise Methylmethacrylat, Ethylmethacrylat, Isopropylmethacrylat, n-Hexylmethacrylat und 2-Hydroxyethylmethacrylat.

Neuerdings werden häufig auch mehrfunktionelle Ester der Methacrylsäure mit höherem Molekulargewicht eingesetzt, wie Ethylenglykoldimethacrylat, Butandiol-1,4-dimethacrylat, Triethylenglykoldimethacrylat, Dodecandiol-1,12-dimethacrylat, Decandiol-1,10-dimethacrylat, 2,2-Bis[p[$\gamma$-methacryloxy-$\beta$-hydroxypropoxy)-phenyl]-propan das Diadukt aus Hydroxyethylmethacrylat und Trimethylhexamethylendiisocyanat, das Diadukt aus Hydroxyethylmethacrylat und Isophorondiisocyanat, Trimethylolpropantrimethacrylat, Pentaerythrittrimethacrylat, Pentaerythrittetramethacrylat und 2,2-Bis[p($\beta$-hydroxy-ethoxy)-phenyl]-propandimethacrylat (Bis-GMA).

Die Materialien für die dentale Anwendung können, je nach Anwendungszeck, auf unterschiedliche Weise ausgehärtet werden. Zahnfüllungsmaterialien gibt es sowohl als lichthärtende als auch als selbsthärtende (autopolymerisierende) Massen. Die lichthärtenden Massen enthalten Photoinitiatoren wie Benzoinalkylether, Benzilmonoketale, Acylphosphinoxide oder aliphatische und aromatische 1,2-Diketo-Verbindungen wie beispielsweise Campherchinon sowie Polymerisationsbeschleuniger wie aliphatische oder aromatische tertiäre Amine (z.B. N,N-Dimethyl-p-toluidin Triethanolamin) oder organische Phosphite und erhärten bei Bestrahlung mit UV- oder sichtbarem Licht.

Die selbsthärtenden Materialien bestehen in der Regel aus einer Katalysator- und einer Basispaste, von denen jede den Bestandteil eines Redoxsystems enthält und die beim Vermischen beider Komponenten polymerisieren. Der eine Bestandteil des Redoxsystems ist meistens ein Peroxid, wie beispielsweise Dibenzoylperoxid, der andere meistens ein tertiäres aromatisches Amin, wie beispielsweise N,N'-Dimethyl-p-toluidin.

Andere Dentalmaterialien wie Prothesenkunststoffe oder Kunststoffmassen zur Herstellung künstlicher Zähne können unter Wärmeeinwirkung polymerisiert werden. Als Initiatoren dienen hier in der Regel Peroxide wie Dibenzoylperoxid, Dilaurylperoxid oder Bis(2,4-dichlor-benzoylperoxid).

Dentalmaterialien enthalten weiterhin in der Regel Pigmente, die - in geringer Menge zugesetzt - dazu dienen, die Farbe der Dentalmassen mit den verschiedenen Schattierungen natürlicher Zähne in Übereinstimmung zu bringen. Geeignete Pigmente sind beispielsweise Eisenoxidschwarz, Eisenoxidrot, Eisenoxidgelb, Eisenoxidbraun, Cadmiumgelb und -orange, Zinkoxid und Titandioxid.

Dentalmaterialien enthalten ferner meist organische oder anorganische Füllstoffe. Dies geschieht, um die Volumenschrumpfung der Kunststoffmasse bei der Polymerisation zu vermindern.

Reines monomeres Methylmethacrylat schrumpft beispielsweise bei der Polymerisation um ca. 20 Vol.%. Durch Zusatz von etwa 60 Gewichtsteilen festem Methylmethacrylat-Perlpolymerisat kann die Schrumpfung auf ca. 5 - 7 Vol.% reduziert werden (DE-PS 24 03 211).

Andere organische Füllstoffe werden erhalten, indem man ein Polymerisat herstellt, das im wesentlichen aus Estern der Methacrylsäure besteht und unvernetzt oder vernetzt ist. Gegebenenfalls enthält dieses Polymerisat oberflächenbehandelte Füllstoffe. Ist es als Polymerisat hergestellt, kann es dem Dentalmaterial in dieser Form zugesetzt werden; ist es dagegen durch Substanzpolymerisation in kompakter Form hergestellt, so muß es vor der Einbringung in das Dentalmaterial erst zu einem sog. Splitterpolymerisat vermahlen werden.

Häufig verwendete vorgebildete Polymerisate sind neben den bereits erwähnten füllstoffhaltigen Perl- und Splitterpolymerisaten Homopolymerisate des Methacrylsäuremethylesters oder, vorzugsweise unvernetzte, Copolymerisate des Methacrylsäuremethylesters mit einem geringen Anteil an Estern der Methacrylsäure oder Acrylsäure mit 2 bis 12 C-Atomen in der Alkoholkomponente, zweckmäßigerweise in der Form eines Perlpolymerisates. Andere geeignete Polymerisate sind unvernetzte Produkte auf der Basis von Polyurethanen, Polycarbonaten, Polyestern und Polyethern.

Anorganische Füllstoffe sind beispielsweise gemahlene Gläser oder Quarz mit mittleren Teilchengrößen zwischen etwa 1 und 10 µm sowie hochdisperses $SiO_2$ mit mittleren Teilchengrößen zwischen etwa 10 - 400 nm.

Bei den Gläsern handelt es sich vorzugsweise um Aluminiumsilikatgläser, die mit Barium, Strontium oder seltenen Erden dotiert sein können (DE-PS 24 58 380).

Hinsichtlich des feingemahlenen Quarzes bzw. der feingemahlenen Gläser sowie des hochdispersen $SiO_2$ bleibt anzumerken, daß der anorganische Füllstoff in der Regel vor dem Vermischen mit den Monomeren zwecks besserer Bindung an die organische Matrix silanisiert wird. Hierfür werden die anorganischen Füllstoffe mit Silankupplungsmitteln überzogen, die meistens eine polymerisierbare Doppelbindung zur Reaktion mit den monomeren Estern der Methacrylsäure aufweisen.

Geeignete Silankupplungsmittel sind beispielsweise Vinyltrichlorsilan, Tris-(2-methoxyethoxy)-vinylsilan, Tris-(acetoxy)-vinylsilan und 3-Methacryloyloxy-propyltrimethoxysilan.

Auch die eingangs erwähnten, neuerdings verwendeten Monomeren mit höherem Molekulargewicht bewirken ebenfalls eine Verringerung der Polymerisationsschrumpfung. Diesen Monomeren werden nun bis zu etwa 85 Gew.% die beschriebenen inerten anorganischen feingemahlenen Gläser oder organischen Füllstoffe oder Gemische aus diesen zugesetzt, wodurch eine weitere Reduzierung der Schrumpfung auf ca. 1 Vol.% erreicht werden kann.

Die anorganischen Füllstoffe bewirken nicht nur eine Verminderung der Polymerisationsschrumpfung, sondern darüber hinaus auch eine erhebliche Verstärkung des organischen Polymergefüges.

Diese Verstärkung macht sich in einer Verbesserung der mechanischen Eigenschaften sowie in einer Erhöhung der Abriebsfestigkeit deutlich bemerkbar (R. Janda, Quintessenz 39, 1067, 1243, 1393 (1988). Gute mechanische Eigenschaften und hohe Abriebsfestigkeit sind wichtige Forderungen, denen eine Dentalmasse, die verlorene Zahnhartsubstanz dauerhaft ersetzen soll, gerecht werden muß.

Neben den verstärkenden Eigenschaften müssen die Füllstoffe aber ebenfalls auch anderen Materialparametern gerecht werden. Ein wesentlicher Parameter in diesem Zusammenhang ist die Polierbarkeit. Hochglanzpolierbarkeit ist für Füllungsmaterialien und Kronen- und Brückenmaterialien aus mindestens zwei Gründen von erheblicher Bedeutung:

- Aus ästhetischen Gründen ist vom Füllungsmaterial eine hochglänzende und völlig homogene Oberfläche zu fordern, damit die Füllung vom umgebenden, absolut glatten, natürlichen Zahnschmelz nicht mehr zu unterscheiden ist. Weiterhin muß diese hochglänzende Füllungsoberfläche ihren Charakter auch langfristig beibehalten.
- Eine hochglatte Füllungsoberfläche ist auch deshalb wichtig, damit Plaque oder verfärbende Medien keine mechanischen Verankerungsstellen vorfinden.

Nun hat es sich aber gezeigt, daß die vorstehend beschriebenen feingemahlenen Quarz- oder Glasfüllstoffe zwar gute verstärkende Eigenschaften besitzen, jedoch hinsichtlich ihrer Polierbarkeit nicht den Anforderungen entsprechen. Daher hat man versucht, diese anorganischen Füllstoffe noch feiner zu mahlen, um homogenere Oberflächen zu erhalten. Den physikalischen Mahlmethoden sind allerdings Grenzen gesetzt, so daß mittlere Korngrößen unter 1 µm nur noch sehr schwer zu erzeugen sind.

Als man hochdisperse Kieselsäure (mittlere Teilchengröße 10 - 400 nm) als Füllstoff in Dentalmassen verwendete (DE-PS 24 03 211), zeigte sich überraschenderweise, daß mit diesen Füllstoffen eine erhebliche Verbesserung der Polierbarkeit erreicht werden konnte. Nachteile der hochdispersen Kieselsäure bestehen aufgrund ihrer starken verdickenden Wirkung, so daß heute in der Regel Füllgrade über 52 Gew.% nicht erreicht werden können, es sei denn, man begnügt sich mit unzureichenden verarbeitungstechnischen Eigenschaften.

Darüber hinaus zeigen die mit hochdisperser Kieselsäure gefüllten Materialien deutlich geringere Festigkeiten und Härten als die mit Ouarz oder feingemahlenen Gläsern gefüllten.

In den deutschen Offenlegungsschriften DE 39 13 250 A1 und DE 39 13 252 A1 sowie in der deutschen Patentschrift DE 39 03 407 werden neue Füllstoffe beschrieben, die in entsprechenden Dentalmaterialien eingesetzt, gute mechanische Eigenschaften bei gleichzeitiger Polierbarkeit gewährleisten. Diese Produkte weisen deshalb in dieser Hinsicht bereits recht befriedigende Eigenschaften auf. Ein Nachteil dieser Systeme ist jedoch darin zu sehen, daß sie häufig unter einer zu geringen Transparenz leiden. Eine erforderliche Farbabstimmung ist daher in diesen Fällen nur schwer möglich und die überwiegend praktizierte Lichthärtung nur unbefriedigend durchführbar.

Es wurde nun gefunden, daß der Einbau von Aluminiumoxideinheiten in das in den zitierten Patentanmeldungen beschriebene Siloxangerüst zu einer deutlichen Verbesserung der Transparenz entsprechender

Dentalmaterialien führt. Voraussetzung dafür ist gleichzeitig die Beachtung einer bestimmten Verfahrensweise bei der Herstellung dieser Füllstoffe, die ebenfalls Gegenstand der Erfindung ist. Ein wichtiges Merkmal ist dabei eine, der eigentlichen Totalkondensation vorgeschaltete Vorkondensation der monomeren Komponenten unter wasserfreien Bedingungen, in Gegenwart eines sauren oder basischen Kondensationskatalysators.

Aufgabe der Erfindung ist, neue licht-, heiß- oder selbsthärtende Dentalmaterialien aus einem polymerisierbaren organischen Bindemittel und einem feinteiligen Füllstoff bereitzustellen, die einerseits auf Hochglanz polierbar sind und damit den ästhetischen Anforderungen eines Dentalwerkstoffes genügen, andererseits aber bessere physikalische Eigenschaften besitzen als die den momentanen Stand der Technik repräsentierenden polierbaren Dentalmaterialien und weiterhin eine stets ausreichende Transparenz haben.

Diese Aufgabe wurde dadurch gelöst, daß ein neues pastöses, in Gegenwart eines Initiators zu einer auf Hochglanz polierbaren Masse aushärtbares Dentalmaterial aus einem polymerisierbaren organischen Bindemittel und einem feinteiligen Füllstoff entwickelt wurde, das als Füllstoff ein Al-haltiges Organopolysiloxan enthält, das aus Einheiten der Formel

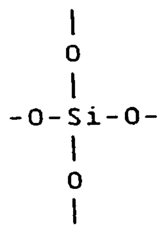

(I)

und Einheiten der Formel

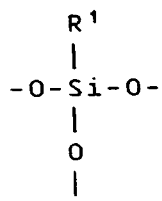

(II)

wobei $R^1$ für eine mit einem Acrylat- oder Methacrylatrest verbundene lineare, gegebenenfalls verzweigte Alkylgruppe mit 1 bis 6 C-Atomen oder für einen einfach olefinisch ungesättigten, vorzugsweise endständig ungesättigten linearen, gegebenenfalls verzweigten Kohlenwasserstoffrest mit 2 bis 8 C-Atomen oder für einen zyklischen, einfach olefinisch ungesättigten Kohlenwasserstoffrest mit 5 - 8 C-Atomen oder für eine lineare, gegebenenfalls verzweigte Alkylgruppe mit 1 bis 8 C-Atomen, eine Phenylgruppe, eine Cycloalkylengruppe mit 5 bis 8 C-Atomen oder eine Alkylarylgruppe steht und/oder Einheiten der Formel

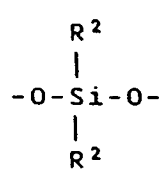

(III)

in denen $R^2$ eine Methyl-, Ethyl- Propyl- oder Phenylgruppe repräsentiert, und - bei jeder der Zusammensetzungen - Einheiten der Formel

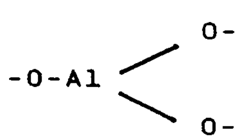

oder

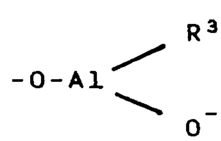

(IV)

in denen $R^3$ eine lineare oder verzweigte Alkylgruppe mit 1 bis 5 C-Atomen oder eine Phenylgruppe ist, vorliegen und die freien Valenzen der an die Silicium- und Aluminiumatome gebundenen Sauerstoffatome bei den Einheiten (I), (II) und/oder (III) sowie (IV) wie bei Heterosiloxangerüsten durch ein Siliciumatom einer gleichen oder einer unterschiedlichen Einheit oder durch ein Aluminiumatom abgesättigt werden, wobei das Verhältnis der Siliciumatome aus den Einheiten der Formel (I) zu der Summe der Siliciumatome der Einheiten (II) und (III) 3 : 1 bis 100 : 1 und das Verhältnis der Summe der Siliciumatome aus den Einheiten (I), (II), und (III) zu den Aluminiumatomen aus den Einheiten (IV) 2 : 1 bis 200 : 1 beträgt.

Die Einheiten nach Formeln (I) bis (IV) können natürlich in unterschiedlicher Form zueinander vorliegen, d. h. sie können in Form eines statistischen Copolykondensates oder in Form eines Block-Copolykondensates oder in Form eines sog. gemischten Copolykondensates vorliegen. Erfindungsgemäß können die Füllstoffe der neuen Dentalmaterialien in bezug auf die Einheiten nach Formel (I) bis (IV) in jeder der genannten Formen und ihrer Gemische vorliegen.

Dies bedeutet, daß im Fall eines reinen statistischen Copolykondensates, das Einheiten der Formel (I), (II), und/oder (III) sowie (IV) enthält, eine rein statistische Verteilung der Komponenten entsprechend der molaren Verhältnisse der Ausgangsprodukte gegeben ist.

Im Fall eines sog. Block-Copolykondensates liegt eine Bildung von Blöcken gleicher Einheiten nach Formel (I) und (II) und/oder (III) sowie (IV) vor. Schließlich weist ein sog. gemischtes Copolykondensat sowohl Strukturen eines statistischen Copolykondensates als auch eines Block-Copolykondensates auf.

Die erfindungsgemäßen Füllstoffe werden in den Dentalmassen in einer Menge von 20 bis 90 Gew.%, vorzugsweise 25 bis 80 Gew.%, eingesetzt. Natürlich können die erfindungsgemäßen Füllstoffe auch in Kombination mit anderen Füllstoffen, wie z. B. Kieselsäure oder feingemahlene Gläser, eingesetzt werden. Der an den Einheiten nach Formel (II) gegebenenfalls vorhandene ungesättigte organische Rest $R^1$ kann vor allem dazu dienen, eine festere Anbindung des Polysiloxan-Füllstoffs an die später aus dem polymerisierbaren organischen Bindemittel erzeugte Polymermatrix zu erreichen.

Besonders geeignet sind deshalb organische Reste $R^1$, bei denen eine Doppelbindung sterisch leicht zugänglich und vor allem auch relativ leicht zu polymerisieren ist. Dies trifft insbesondere zu für die Gruppe

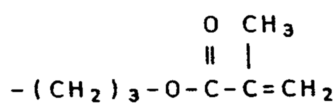

weil deren besonders leichte Polymerisierbarkeit bekannt ist und es sich daneben bei der Polymermatrix, in die der Füllstoff einzubringen ist, in der Regel um ein Methacrylatsystem handelt sowie für lineare Kohlenwasserstoffreste mit endständiger Doppelbindung, wie z. B. dem Vinyl-, Butenyl- oder Octenylrest. Aber auch zyklische Kohlenwasserstoffreste mit polymerisierbaren Doppelbindungen sind geeignet. In vielen Fällen kann $R^1$ aber eine der ebenfalls unter Formel (II) in Anspruch 1 genannten doppelbindungsfreien organischen Reste sein.

Eine besonders vorteilhafte Füllstoff-Zusammensetzung, die sich durch einfache Realisierbarkeit und vor allem durch die technische Verfügbarkeit der Ausgangsmaterialien auszeichnet, sieht vor, daß ein Organopolysiloxan als Füllstoff verwendet wird, das nur aus den Einheiten der Formel (I) und den speziellen Einheiten der Formel (II)

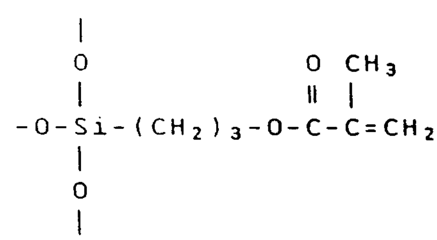

sowie Einheiten der Formel (IV) besteht, wobei das molare Verhältnis der Einheiten der Formel (I) zu den Einheiten der Formel (II) 3 : 1 bis 100 : 1 und das Verhältnis der Summe der Siliciumatome aus den Einheiten (I) und (II) zu den Aluminiumatomen aus Einheiten (IV) 2 : 1 bis 200 : 1 beträgt. Ein derartiger Füllstoff zeichnet sich dadurch aus, daß er zur Einbringung in die Methacrylatmatrix prinzipiell nicht mehr

silanisiert, d.h. mit einem Methacrylsilan behandelt werden muß, nachdem diese Methacrylgruppen bereits in dem Füllstoff homogen verteilt vorhanden sind.

Dies schließt jedoch nicht aus, daß im Einzelfall, im Hinblick auf eine weitere Hydrophobierung mit weiterer Verstärkung der Anbindung zwischen dem Organopolysiloxan-Füllstoff und organischer Polymermatrix, zusätzlich eine Silanisierung des Füllstoffs vorgenommen wird.

Bei der Ausarbeitung der Erfindung hat sich herausgestellt, daß sehr gute mechanische Eigenschaften und Polierbarkeit des Dentalmaterials auch dann erreicht werden können, wenn der verwendete Organosiloxan-Füllstoff keine ungesättigten, sondern nur gesättigte Reste $R^1$ enthält.

Dies gilt sowohl für Füllstoffe, die Einheiten nach Formel (I), (II) und (III) und (IV) als auch für solche Füllstoffe, die nur Einheiten nach Formel (I) und (II) enthalten. Derartige nicht-doppelbindungsfunktionelle Organopolysiloxan-Füllstoffe sollten vor ihrer Einbringung in die organische Polymermatrix mit einer geeigneten Organosilanverbindung, vorzugsweise 3-Methacryloyloxy-propyltrimethoxy- oder 3-Methacryloyloxypropyltriethoxysilan, behandelt werden.

Analoges gilt auch für eine, aus Gründen der besonders leichten Verfügbarkeit der Ausgangsmaterialien vorteilhafte erfindungsgemäße Füllstoffzusammensetzung, die einen Aufbau des Polysiloxans aus Einheiten der Formel (I) und den speziellen Einheiten der Formel (III)

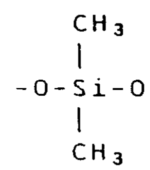

sowie Einheiten der Formel (IV) vorsieht, wobei das molare Verhältnis der Einheiten nach Formel (I) zu den Einheiten der Formel (III) 3 : 1 bis 100 : 1 und das Verhältnis der Summe der Siliciumatome aus den Einheiten (I) und (II) zu den Aluminiumatomen aus Einheiten (IV) 2 : 1 bis 200 : 1 beträgt.

Weiterhin gilt für alle beanspruchten erfindungsgemäßen Ausführungen, daß der im Hinblick auf die gewünschte Transparenz der mit den Füllstoffen hergestellten Dentalmaterialien notwendige Anteil an Aluminium so zu wählen ist, daß das Verhältnis der Summe der Si-Atome aus den Formeln (I), (II) und (III) zu den Aluminiumatomen aus den Einheiten (IV) 2 : 1 bis 200 : 1 beträgt.

Die monomeren Bausteine der erfindungsgemäßen Füllstoffe sind prinzipiell bekannte Verbindungen, beispielsweise $Si(OC_2H_5)_4$ als Monomerbaustein für eine Einheit der Formel (I), eine Verbindung

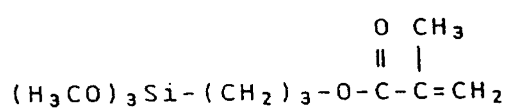

bzw.

$(H_3CO)_3Si\text{-}CH_2CH_2CH_3$

als Monomerbausteine für Einheiten der Formel (II) und eine Verbindung $(H_3C)_2Si(OC_2H_5)_2$ als Monomerbaustein für Einheiten der Formel (III) sowie eine Verbindung $Al(OC_2H_5)_3$ als Monomerbaustein für Einheiten der Formel (IV).

Die Zusammensetzungen der daraus herstellbaren erfindungsgemäßen Dentalfüllstoffe lassen sich z.B. durch Formeln für eine jeweilige Polymereinheit wie

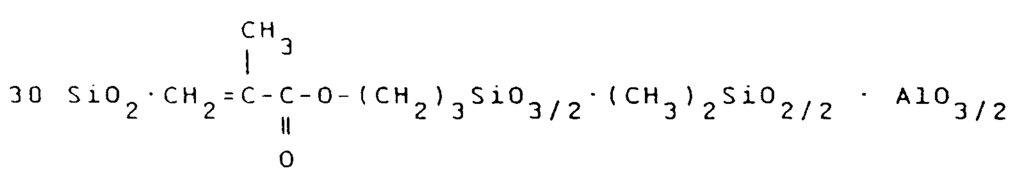

$10\ SiO_2 \cdot C_3H_7SiO_{3/2} \cdot (H_5C_2)_2SiO_{2/2} \cdot 2\ AlO_{3/2}$

$50\ SiO_2 \cdot (H_3C)_2SiO_{2/2} \cdot 6\ AlO_{3/2}$ oder

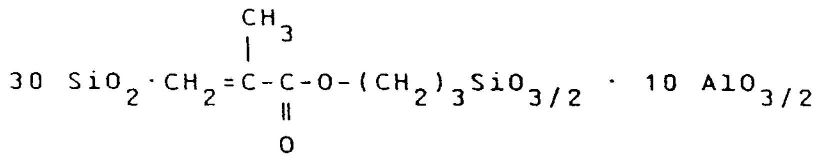

beschreiben. Einheiten der Formel (II) gemäß Anspruch 4 und Einheiten der Formel (III) gemäß Anspruch 5 sind u. a. auch deshalb bevorzugt, weil die entsprechenden Monomeren technisch verfügbare Produkte darstellen.

Im Hinblick auf die physikalischen Eigenschaften besitzen Füllstoffe der erfindungsgemäßen Zusammensetzung dann eine besonders gute Eignung zur Verwendung in den erfindungsgemäßen Dentalmaterialien, wenn sie eine spezifische Oberfläche von ca. 10 bis 250 $m^2/g$, vorzugsweise ca. 30 bis 200 $m^2/g$, und eine Partikelgröße von 0,01 $\mu m$ bis 100 $\mu m$, vorzugsweise 0,1 $\mu m$ bis 30 $\mu m$, aufweisen.

Die auf die Erzielung eines statistischen Copolykondensats gerichtete Herstellungsmethodik der erfindungsgemäßen Dentalfüllstoffe sieht vor, daß man ein Alkoxysilan der allgemeinen Formel

$Si(OR^4)_4$ (V)

und ein Alkoxysilan der allgemeinen Formel

$R^1 - Si(OR^4)_3$ (VI)

in der $R^1$ dieselbe Bedeutung wie in Formel (II) hat, und/oder ein Alkoxysilan der allgemeinen Formel

$(R^2)_2Si(OR^4)_2$ (VII)

in der $R^2$ dieselbe Bedeutung wie in Formel (III) hat, sowie eine Aluminiumverbindung der allgemeinen Formel

$Al(OR^4)_3$ bzw. $AlR^3(OR^4)_2$ (VIII)

in der $R^3$ dieselbe Bedeutung wie in Formel (IV) hat, wobei $R^4$ für eine lineare oder verzweigte Alkylgruppe mit 1 bis 5 C-Atomen steht, in einem weitgehend wassermischbaren, aber die Verbindungen nach Formel (V), (VI), (VII) sowie (VIII) lösenden Lösungsmittel auflöst, dann das Reaktionsgemisch in Gegenwart eines sauren, basischen oder Metall-haltigen Katalysators unter Rühren bei einer bestimmten Temperatur im Bereich von Raumtemperatur bis 200° C vorkondensiert, durch Zusatz von gegebenenfalls Säure- oder Base-haltigem Wasser die Hydrolyse und vollständige Polykondensation durchführt und den gebildeten Feststoff, gegebenenfalls nach Zusatz weiteren Lösungsmittels oder Wassers noch 1 Stunde bis zu 6 Stunden bei 60° C bis 200° C, bei Normaldruck oder einem Druck rührt, welcher der Summe der Partialdrucke bei der jeweiligen Temperatur entspricht, dann das gebildete Organopolysiloxan, gegebenenfalls nach einem Wechsel des Mediums und/oder pH-Wertes noch 1 Stunde bis 5 Tage bei 60° C bis 250° C in flüssiger Phase nachbehandelt, dann nach gängigen Techniken von der flüssigen Phase abtrennt, gegebenenfalls wäscht, bei Raumtemperatur bis 200° C, gegebenenfalls unter Schutzgasatmosphäre oder im Vakuum trocknet, gegebenenfalls anschließend 1 bis 100 Stunden bei Temperaturen von 150° C bis 250° C unter Schutzgasatmosphäre oder im Vakuum tempert, gegebenenfalls mahlt und/oder klassifiziert, wobei man das von der flüssigen Phase abgetrennte und gegebenenfalls gewaschene Organopolysiloxan vor oder nach einer der Stufen Trocknung, Temperung, Mahlung, Klassifizierung in Wasser, einem Wasser/Alkohol-Gemisch oder in reinem Alkohol, in Gegenwart eines sauren oder eines basischen Katalysators, bevorzugt in Gegenwart von Ammoniak oder Alkali- oder Erdalkalioxiden bzw. -hydroxiden, über einen Zeitraum von 1 Stunde bis zu 5 Tagen bei Temperaturen von 60° C bis 250° C unter einem Druck, welcher der Summe der Partialdrucke bei der jeweiligen Temperatur entspricht, behandelt und vor der weiteren Aufarbeitung säure- oder laugefrei wäscht.

Nach einer günstigen Ausführungsform der Vorkondensation wird so vorgegangen, daß man die monomeren Komponenten nach Formel (V), (VI) und/oder (VII) sowie (VIII) ohne oder unter Verwendung eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer weitgehend wasserfreien Säure oder Base über einen Zeitraum von 5 Minuten bis zu 5 Tagen bei Raumtemperatur bis 200 ° C vorkondensiert.

Ein typischer saurer Katalysator ist zum Beispiel Salzsäure oder Essigsäure oder eine Alkylsulfonsäure oder auch Lewissäure, während zum Beispiel neben Ammoniak Amine und andere Lewisbasen oder Alkali- oder Erdalkalialkoholate typische Basenkatalysatoren repräsentieren.

Mit Hilfe dieser Katalysatoren wird die Vorkondensation unter weitgehend wasserfreien Bedingungen durchgeführt. Dabei findet eine Äquilibrierung in bezug auf die Alkoxygruppen an den verschiedenen Monomerkomponenten nach Formel (V) bis (VIII) und eine Oligomerenbildung statt. Die Vorkondensation ist somit Voraussetzung, um ein homogenes Gelierverhalten der Komponenten nach Formel (V) bis (VIII) und ein einheitliches Polykondensat zu erreichen. Dieser Aspekt ist besonders wichtig in bezug auf die Aluminiumkomponente nach Formel (VIII), weil diese eine deutlich höhere Hydrolyse- und Kondensationsbereitschaft als die Silankomponenten (V) bis (VII) aufweist. Um die Vorkondensation nicht durch eine vorzeitige zu weitgehende Kondensation einer Komponente zu stören, ist dabei die Anwesenheit von Wasser unerwünscht. also weitgehende Wasserfreiheit erforderlich. Überraschenderweise liefern nur so hergestellte Füllstoffe gemäß der Erfindung in Dentalmaterialien eine ausreichende Transparenz bei gleichzeitig hervorragenden mechanischen Eigenschaften und guter Polierbarkeit.

Die Dauer der Vorkondensation und die angewandte Reaktionstemperatur hängen in der Regel von der Reaktivität der Monomerkomponenten nach Formeln (V) bis (VIII) ab, die generell im Fall des Aluminiums höher ist als im Fall des Siliciums. Bei den Si-haltigen Monomerkomponenten (V), (VI) und (VII) hängt die Reaktivität insbesondere von den Gruppen $R^4$ und den Substituenten $R^1$ bzw. $R^2$ in der Weise ab, daß mit zunehmender Größe derselben eine Abnahme der Reaktivität auftritt.

Die vorteilhaften anwendungstechnischen Eigenschaften der neuen Füllstoffe gehen auch auf die saure oder alkalische Temperaturbehandlung vor oder nach Trocknung oder in einer der gegebenenfalls noch angewandten Behandlungsstufen zurück, da dadurch vor allem eine Festigung der Polymerstruktur erreicht wird.

Prinzipiell können als Ausgangsstoffe für das Verfahren anstelle der Alkoxyverbindungen auch die entsprechenden Halogenid- oder Phenoxyverbindungen eingesetzt werden, doch bietet deren Verwendung keine Vorteile, sondern kann zum Beispiel im Fall der Chloride, Schwierigkeiten durch die bei der Hydrolyse freiwerdende Salzsäure verursachen.

Die Hydrolyse der Vorkondensate muß in einem weitgehend wassermischbaren, aber die Ausgangsstoffe lösenden Lösungsmittel durchgeführt werden. Bevorzugt werden Alkohole verwendet, die zu den Alkoxygruppierungen an den monomeren Ausgangsstoffen korrespondieren.

Besonders geeignet sind Methanol, Ethanol, n- und i-Propanol, n- und i-Butanol und n-Pentanol. Auch Gemische solcher Alkohole können als Lösungsmittel bei der Hydrolyse eingesetzt werden.

Anstelle von Alkoholen können natürlich auch andere polare Lösungsmittel, die weitgehend wassermischbar sind, verwendet werden, doch erweist sich dies aus verfahrenstechnischen Gründen wegen der mit dem hydrolytisch abgespaltenen Alkohol zustandekommenden Lösungsmittelgemische als nicht sinnvoll.

Bevorzugt führt man die Hydrolyse mit einem Überschuß an Wasser über die stöchiometrisch erforderliche Menge durch. Die zur Hydrolyse benötigte Menge Wasser hängt von der Hydrolysegeschwindigkeit des Vorkondensats in der Weise ab, daß mit zunehmender Menge Wasser raschere Hydrolyse erfolgt, allerdings kann eine Obergrenze durch auftretende Entmischung und Ausbildung eines Zweiphasensystems vorgegeben sein. Grundsätzlich ist eine Hydrolyse in homogener Lösung vorzuziehen.

Aufgrund der genannten Aspekte wird in der Praxis gewichtsmäßig maximal die Menge Wasser verwendet, wie an Silanmonomeren insgesamt eingesetzt wird.

Das zur Hydrolyse verwendete Wasser kann eine organische oder anorganische Säure oder Base enthalten. Der Zusatz von Säuren oder Base kann einerseits unter dem Aspekt der Neutralisation der Reaktionsmischung nach saurer oder basischer Vorkondensation oder im Hinblick auf die Einstellung eines optimalen pH-Werts bei der Gelierung erfolgen.

So kann z. B. die Vorkondensation unter sauren und die Gelierung unter basischen Bedingungen durchgeführt werden.

Die Polykondensation kann bei verschiedenen Temperaturen durchgeführt werden. Nachdem die polykondensation bei höheren Temperaturen am schnellsten verläuft, wird diese bevorzugt bei Rückflußtemperatur oder knapp darunter durchgeführt. Prinzipiell kann die Hydrolyse und Polykondensation bei noch höherer Temperatur als Rückflußtemperatur, d. h. unter Druck, durchgeführt werden.

Bei der Polykondensation kann das Reaktionsgemisch zu einer festen Masse erstarren. Aus diesem Grunde ist es angebracht, eine entsprechende Menge Lösungsmittel oder Wasser zur Verdünnung

zuzusetzen.

Das Lösungsmittel wird dabei in der Regel dasselbe sein, das schon bei der Hydrolyse der Silane eingesetzt wurde, d. h. bevorzugt wird ein niederer Alkohol mit 1 bis 5 C-Atomen verwendet.

Alternativ zu der Verdünnung mit einem Lösungsmittel kann natürlich auch mit Wasser verdünnt werden. Was im Einzelfall verwendet wird, hängt auch davon ab, welche physikalischen Eigenschaften das herzustellende Copolykondensat haben soll.

Auch durch die Dauer und Temperatur der Nachbehandlung in flüssiger Phase oder in trockener Form kann hierauf Einfluß genommen werden. Eine Nachreaktion bei höherer Temperatur führt durchwegs zu einer Verfestigung der Struktur des Polymeren und zu einer Verbesserung der mechanischen Eigenschaften.

Die Abtrennung des gebildeten Feststoffs kann nach gängigen Techniken wie Filtrieren, Dekantieren, Zentrifugieren oder durch Abdestillieren der flüssigen Phase erfolgen. Die Waschung des gebildeten Feststoffs wird bevorzugt mit dem bei der Fällung verwendeten Lösungsmittel oder mit Wasser durchgeführt.

Das getrocknete bzw. getemperte Produkt kann in üblichen Vorrichtungen gemahlen und in verschiedene Korngrößenfraktionen klassifiziert werden. Von den Aufarbeitungsmaßnahmen Waschung, Trocknung, Temperung, Mahlung und Klassifizierung kann, je nach Umständen, die eine oder andere entfallen oder in einer anderen Reihenfolge durchgeführt werden.

Eine Klassifizierung kann zum Beispiel auch an flüssigkeitsfeuchtem, gegebenenfalls vorher getrocknetem oder getempertem Produkt durchgeführt werden.

Die Dauer der Hydrolyse hängt von der Hydrolysenneignung der Vorkondensate und von der Temperatur ab. Die Hydrolysegeschwindigkeit hängt insbesondere von den Silicium-ständigen Alkoxygruppen ab, wobei die Methoxygruppe am schnellsten hydrolysiert und mit steigender Kettenlänge oder zunehmender Verzweigung eine Verlangsamung eintritt.

Gemäß Anspruch 7 werden statistische Copolykondensate erhalten.

Nach einer anderen Methode gemäß Anspruch 10 werden sog. Block-Copolykondensate erhalten, bei denen eine Bildung von Blöcken gleicher Einheiten nach Formel (I) und (II) und/oder (III) sowie (IV) vorliegt. Dieses Verfahren sieht vor, daß man die monomeren Komponenten nach Formel (V), (VI) und/oder (VII) sowie (VIII) jeweils unabhängig voneinander oder in einer Kombination von jeweils 2 oder aber maximal 3 Komponenten, ohne oder unter Verwendung eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer wasserfreien Säure oder Base über einen Zeitraum von 5 Minuten bis zu 5 Tagen bei Raumtemperatur bis 200° C vorkondensiert, die erhaltenen Kondensate vereinigt, dann gemeinsam über einen Zeitraum von 5 Minuten bis zu 2 Tagen bei Raumtemperatur bis 200° C nochmals vorkondensiert und hierauf nach Zusatz von gegebenenfalls Säure- oder Base-haltigen Wassers und gegebenenfalls nach Zusatz weiteren Lösungsmittels die Hydrolyse und vollständige Polykondensation, wie schon bei den statistischen Copolykondensaten beschrieben, durchführt.

Nach einer weiteren Methode werden sog. gemischte Copolykondensate erhalten, bei denen teilweise eine Bildung von Blöcken gleicher Einheiten nach Formel (I) und (II) und/oder (III) sowie (IV) vorliegt, bei denen stets jedoch mindestens eine monomere Komponente zunächst nicht vorkondensiert und mindestens eine monomere Komponente zunächst für sich oder in Kombination mit anderen Komponenten vorkondensiert werden.

Um keine Differenzen im Gelierverhalten von vorkondensierten Komponenten und nicht vorkondensierten Komponenten hervorzurufen, ist nach deren Vereinigung nochmals eine gemeinsame Vorkondensation erforderlich.

Das Verfahren zur Gewinnung gemischter Copolykondensate sieht vor, daß man von den monomeren Komponenten nach Formel (V), (VI) und/oder (VII) sowie (VIII) mindestens ein Monomer aber höchstens 3 Monomere voneinander unabhängig oder in Kombination miteinander, ohne oder unter Verwendung eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer weitgehend wassserfreien Säure oder Base, über einen Zeitraum von 5 Minuten bis zu 5 Tagen bei Raumtemperatur bis 200° C vorkondensiert, das erhaltene Vorkondensat bzw. die erhaltenen Vorkondensate und mindestens eine nicht vorkondensierte Komponente miteinander vereinigt, die vereinigten Komponenten dann über einen Zeitraum von 5 Minuten bis zu 2 Tagen bei Raumtemperatur bis 200° C nochmals vorkondensiert und dann nach Zusatz von gegebenenfalls Säure- oder Base-haltigen Wassers und gegebenenfalls weiteren Lösungsmittels die Hydrolyse und vollständige Polykondensation wie bei den vorstehend beschriebenen beiden Methoden durchführt.

Die Verwendung eines metallhaltigen Kondensationskatalysators zur Vorkondensation ist auch bei dieser Herstellungsvariante prinzipiell möglich und die weitere Behandlung des gebildeten Polykondensates gestaltet sich ebenso wie bei den anderen beschriebenen Herstellungsverfahren.

Die Dauer der Vorkondensation hängt, wie bereits vorstehend beschrieben, generell natürlich auch von der Reaktivität der monomeren Komponenten und der Temperatur ab.

Charakterisiert sind die Füllstoffe für die neuen Dentalmaterialien insbesondere anhand der quantitativen Hydrolyse- und Kondensationsausbeute und der Elementaranalysen. Zwischen den nach den unterschiedlichen Herstellungsverfahren erhaltenen Copolykondensaten besteht rein optisch kein Unterschied.

Je nach Behandlung besitzen die erfindungsgemäßen Füllstoffe Oberflächen 10 bis 250 $m^2/g$, vorzugsweise 30 bis 200 $m^2/g$. Die gewünschten Teilchengrößendurchmesser von 0,01 $\mu m$ bis 100 $\mu m$ lassen sich problemlos durch Anwendung gängiger Mahltechniken einstellen.

Ein weiterer Gegenstand der Erfindung ist die Verwendung des Dentalmaterials nach vorstehenden Ansprüchen zur Fertigung von Zahnfüllungen, Inlays, Zahnversiegelungen, Überzügen zum Schutz von Zahnoberflächen, Kronen, Verblendungen, Brücken, Zahnprothesen, künstlichen Zähnen, Klebern zur Befestigung von Inlays, Kronen und Brücken sowie zum Aufbau von Zahnstümpfen.

Die Erfindung wird nachfolgend anhand von Ausführungsbeispielen weiter erläutert.

I. Herstellung der erfindungsgemäßen Füllstoffe

Beispiel 1

1379,8 g (6,62 Mol) $Si(OC_2H_5)4$, 54,8 g (0,22 Mol) Methacryloyloxypropyltrimethoxysilan und 54,4 g (0,22 Mol) $Al(0\text{-sec.}C_4H_9)_3$ wurden unter Rühren mit 113 ml 4n ethanolischer HCl-Lösung binnen 10 Min. versetzt. Die Mischung wurde auf Rückflußtemperatur aufgeheizt und 1 Stunde bei dieser Temperatur gerührt. Anschließend wurden 600 ml Ethanol zugegeben und nochmals 3 Stunden bei dieser Temperatur gerührt. Nach Abkühlen auf 50° C wurden 525 ml 10 %ige wäßrige $NH_3$-Lösung innerhalb von 30 Min. zugegeben. Kurz darauf begann der Ansatz aus der homogenen Lösung heraus zu gelieren. Nach Erhöhung der Rührgeschwindigkeit auf 700 U/Min. wurden der gebildeten Suspension 750 ml entsalztes Wasser zugesetzt. Es wurde noch 1 Stunde unter Rückfluß gerührt, dann abgekühlt und der Feststoff abfiltriert. Dieser wurde in 500 ml 5 %iger Ammoniaklösung eingerührt. Die Suspension wurde noch 24 Stunden in einem Autoklaven bei 150° C unter Eigendruck gerührt. Der Feststoff wurde abfiltriert, mit Wasser neutral gewaschen, dann 24 Stunden bei 120° C getrocknet und 10 Stunden in der Kugelmühle gemahlen, bis der mittlere Teilchendurchmesser im Bereich von 15 $\mu m$ lag.

Es wurden 443 g (99,0 % der Theorie) eines Dentalfüllstoffs, bestehend aus Polmereinheiten der Formel

$$H_2C=\overset{\overset{\displaystyle CH_2}{|}}{C}-\underset{\underset{\displaystyle O}{\|}}{C}-O-(CH_2)_3-SiO_{3/2}\cdot 30\,SiO_2\cdot 1\,AlO_{3/2}$$

erhalten. Alle Elementaranalysen befanden sich in Übereinstimmung mit dieser Zusammensetzung.

| | |
|---|---|
| Spezifische Oberfläche: | 127 $m^2/g$ |

Beispiel 2

950 g (4,56 Mol) $Si(OC_2H_5)_4$, 22,54 g (0,151 Mol) $(H_3C)_2Si(OC_2H_5)_2$ und 224,65 g (0,912 Mol) $Al(0\text{-sec.}C_4H_9)_3$ wurden in einem 3 l-Glasgefäß mit Rührer, Rückflußkühler und Innenthermometer vereinigt. Die Mischung wurde binnen 5 Min. mit 88 ml 4n ethanolischer HCl-Lösung versetzt und 6 Stunden unter Rückfluß gerührt. Dann wurde die Mischung auf 50° C abgekühlt und mit 500 ml Ethanol sowie mit 420 ml 10 %iger wäßriger $NH_3$-Lösung versetzt. Es wurde bei ca. 70° C weiter gerührt, bis nach 5 Min. die Gelierung einsetzte. Dem sich bildenden Gel wurden zur Verdünnung noch 600 ml Wasser zugesetzt. Nach einer weiteren Rückflußphase von 1 Stunde wurde der Ansatz analog zu Beispiel 1 aufgearbeitet, wobei die

Nachbehandlung des abfiltrierten Feststoffs im Autoklaven in 500 ml 2 %iger $NH_3$-Lösung durchgeführt wurde.

Es wurden 328 g (99,5 % der Theorie) eines Dentalfüllstoffs, bestehend aus Polymereinheiten der Formel

$(H_3C)_2SiO_{2/2} \cdot 30\ SiO_2 \cdot 6\ AlO_{3/2}$

erhalten. Die Elementaranalysen befanden sich in Übereinstimmung mit dieser Zusammensetzung.

| | |
|---|---|
| Spezifische Oberfläche: | 93 $m^2/g$ |

Beispiel 3

16,43 g (0,1 Mol) n-$C_3H_7$-$Si(OCH_3)_3$, 264,4 g (1,0 Mol) $Si(OC_3H_7)_4$ und 24,63 g (0,1 Mol) $Al(OC_4H_9)_3$ wurden jeweils mit 2 ml Essigsäure (100 %) versetzt und jeweils 1 Stunde bei 70° C gerührt. Anschließend wurden die 3 Vorkondensate in einem 2 l-Rührkolben mit KPG-Rührer, Rückflußkühler und Innenthermometer vereinigt und nochmals 1 Stunde bei 70° C gerührt. Die Mischung wurde auf 50° C abgekühlt mit 300 ml Isopropanol und dann mit 100 ml 2 %iger wäßriger Ammoniaklösung versetzt und solange unter Rückflußtemperatur weiter gerührt, bis die Gelierung einsetzte. Das sich bildende Gel wurde mit 300 ml Wasser versetzt, noch 2 Stunden unter Rückfluß gerührt und analog zu Beispiel 1 weiter behandelt, mit der Ausnahme, daß die Nachbehandlung im Autoklaven in 200 ml NaOH-Lösung mit pH 10 durchgeführt wurde.

Erhalten wurden 73,5 g (98,4 % der Theorie) eines Dentalfüllstoffs, dessen Zusammensetzung in Übereinstimmung mit den gefundenen Analysenwerten folgender Formel entspricht:

n-$C_3H_7$-$SiO_{3/2} \cdot 10\ SiO_2 \cdot AlO_{3/2}$

| | |
|---|---|
| Spezifische Oberfläche: | 78 $m^2/g$ |

Beispiel 4

17,83 g (0,1 Mol) $H_3C$-$Si(OC_2H_5)_3$, 14,83 g (0,1 Mol) $(CH_3)_2Si(OC_2H_5)_2$, 14,62 g (0,1 Mol) $H_5C_2Al$-$(OC_2H_5)_2$ wurden vereinigt. Die Mischung wurde 6 Stunden unter Rückfluß gerührt, dann mit 416,7 g (2,0 Mol) $Si(OC_2H_5)_4$ versetzt und hierauf binnen 10 Min. mit 200 ml Ethanol verdünnt und danach 200 ml 5 %iger wäßrige $NH_3$-Lösung zugegeben. Es wurde weiter unter Rückfluß gerührt, bis die Gelierung einsetzte. Nach Verdünnung mit 500 ml $H_2O$ wurde noch 2 Stunden unter Rückfluß gerührt und anschließend weiter wie nach Beispiel 1 verfahren.

Erhalten wurden 140,2 g (99,1 % der Theorie) eines Dentalfüllstoffs, dessen Zusammensetzung in Übereinstimmung mit den gefundenen Analysenwerten durch folgende Formel zu beschreiben ist:

$H_3C$-$SiO_{3/2} \cdot (H_3C)_2SiO_{2/2} \cdot 20\ SiO_2 \cdot H_5C_2AlO_{3/2}$

| | |
|---|---|
| Spezifische Oberfläche: | 46 $m^2/g$ |

Beispiel 5

375,0 g (1,8 Mol) $Si(OC_2H_5)_4$, 14,9 g (0,06 Mol) Methacryloyloxypropyltrimethoxysilan und 9,73 g (0,06 Mol) $Al(OC_2H_5)_3$ wurden in einem 2 l Glasgefäß mit KPG-Rührer, Innenthermometer und Rückflußkühler vereinigt. Die Mischung wurde binnen 5 Min. mit 100 ml 10 %iger ethanolischer $NaOC_2H_5$-Lösung versetzt. Die klare Lösung wurde anschließend 2 Stunden unter Rückfluß gerührt und dann binnen 10 Min. mit 100 ml 1 %iger wäßriger $NH_3$-Lösung versetzt. Das sich spontan bildende Gel wurde mit 400 ml Wasser verdünnt und noch 15 Min. unter Rückfluß gerührt, dann abgekühlt und der Feststoff von der flüssigen Phase abfiltriert. Der feuchte Feststoff wurde in genau 2 gleiche Teile geteilt.

a) 1 Teil wurde mit 150 ml wäßriger NaOH-Lösung mit pH 11,7 versetzt und 24 Stunden bei 150° C im Autoklaven unter Eigendruck gerührt.

b) Die 2. Hälfte des Feuchtprodukts wurde mit 150 ml 5 %iger wäßriger $NH_3$-Lösung versetzt und 24 Stunden bei 150° C im Autoklaven unter Eigendruck gerührt.

Nach der Behandlung wurden beide Produkte mit Wasser neutral gewaschen und 24 Stunden unter $N_2$-Atmosphäre bei 120° C getrocknet. Die Gesamtausbeute betrug 119 g (97,6 % der Theorie).

Die gefundenen Analysendaten waren in Übereinstimmung mit der Zusammensetzung

$$CH_2=C(CH_3)-C(=O)-O-(CH_2)_3SiO_{3/2} \cdot 30\ SiO_2 \cdot AlO_{3/2}$$

| Spezifische Oberfläche: | |
|---|---|
| Produkt a) | 152 $m^2/g$ |
| Produkt b) | 136 $m^2/g$ |

Beispiel 6

Ausgehend von 375,0 g (1,8 Mol) $Si(OC_2H_5)_4$, 8,9 g (0,06 Mol) $(CH_3)_2Si(OC_2H_5)_2$ und 14,8 g (0,06 Mol) $Al(OC_4H_9)_3$ wurde genau analog zu Beispiel 5 verfahren. Erhalten wurde ein Produkt der Zusammensetzung

$(CH_3)_2SiO_{2/2} \cdot 30\ SiO_2 \cdot AlO_{3/2}$

in angenähert quantitativer Ausbeute.

| Spezifische Oberfläche: | |
|---|---|
| Produkt a) | 137 $m^2/g$ |
| Produkt b) | 90 $m^2/g$ |

II. Herstellung der erfindungsgemäßen Dentalwerkstoffe

Zur Herstellung der erfindungsgemäßen Dentalmassen wurden die Füllstoffe aus den Beispielen 1, 2, 5, 6 mit einer mittleren Korngröße von 15 µm verwendet. Die Füllstoffe wurden nach üblichen Verfahren mit 3-Methacryloyloxypropyltrimethoxysilan silanisiert. Die Füllstoffe wurden in Mengen von 51 bis 62 % (m/m) in eine Monomermatrix eingebracht, wie sie für Dentalkunststoffe üblicherweise verwendet wird. Es wurden noch Initiatoren zugesetzt, und die Massen wurden zu homogenen Pasten geknetet.

Es wurde eine Reihe von physikalischen Eigenschaften aus den verschiedenen Pasten hergestellten ausgehärteten Prüfkörpern bestimmt und mit denen von Handelsprodukten und Laborvergleichsprodukten verglichen (Tabelle I).

Beispiele für erfindungsgemäße Dentalmassen:

1 . Heißhärtende erfindungsgemäße Dentalmassen:

Die Herstellung der Prüfkörper aus den heißhärtenden erfindungsgemäßen Dentalmassen erfolgte in der Weise, daß die Massen in die entsprechenden Prüfkörperformen gepreßt und dann in einem Wasserbad unter 6 atü Druck bei 90° C 30 Min. lang ausgehärtet wurden.

Beispiel Nr. 15 (Angaben in Gewichtsteilen)

61,5 Füllstoff Nr. 1
14,3 Bis-GMA
11,0 UDMA

11,0 TEDMA
2,2 Dibenzoylperoxid

Beispiel Nr. 16 (Angaben in Gewichtsteilen)

53,0 Füllstoff Nr. 3
4,5 hochdisperses $SiO_2$
22,0 UDMA
8,0 Bis-GMA
10,0 TEDMA
2,5 Dibenzoylperoxid

Beispiel Nr. 17 (Angaben in Gewichtsteilen)

50,4 Füllstoff Nr. 5a
5,0 hochdisperses $SiO_2$
22,6 UDMA
8,3 Bis-GMA
10,4 TEDMA
3,3 Dibenzoylperoxid

Beispiel Nr. 18 (Angaben in Gewichtsteilen)

58,3 Füllstoff Nr. 6a
15,9 Bis-GMA
11,9 UDMA
11,9 TEDMA
2,0 Dibenzoylperoxid

2. Lichthärtende erfindungsgemäße Dentalmassen:

Die lichthärtenden erfindungsgemäßen Dentalmassen bestehen aus transparenten Pasten, die durch Bestrahlung mit einer zahnärztlichen Halogenlichtlampe (Degulux Degussa) ausgehärtet werden. Bestrahlungszeit 100 Sek.

Beispiel Nr. 19 (Angaben in Gewichtsteilen)

48,3 Füllstoff Nr. 1
6,0 hochdisperses $SiO_2$
22,6 UDMA
8,2 Bis-GMA
10,3 TEDMA
4,6 Initiatoren

Beispiel Nr. 20 (Angaben in Gewichtsteilen)

47,9 Füllstoff Nr. 2
4,4 hochdisperses $SiO_2$
23,6 UDMA
8,6 Bis-GMA
10,8 TEDMA
4,7 Initiatoren

Beispiel Nr. 21 (Angaben in Gewichtsteilen)

42,9 Füllstoff Nr. 5b
6,7 hochdisperses $SiO_2$
25,0 UDMA 9,1 Bis-GMA
11,3 TEDMA
5,0 Initiatoren

Beispiel Nr. 22 (Angaben in Gewichtsteilen)

44,6 Füllstoff Nr. 6a
6,5 hochdisperses $SiO_2$
24,3 UDMA
8,8 Bis-GMA
11,0 TEDMA
4,8 Initiatoren

Abkürzungen:

Bis-GMA: 2,2-Bis[p-($\gamma$-methacrylolyoxy-$\beta$-hydroxypropoxy)-phenyl]-propan
UDMA: 7,7,9-Trimethyl-4,13-dioxo-3,14-dioxa-5, 12-diazahexadecan-1,16-dioldimethacrylat

TEDMA: Triethylenglycoldimethacrylat

Handelsprodukte:

Handelsprodukte, mit denen die erfindungsgemäßen Dentalmassen in Tabelle I verglichen werden: Konventionelle Composite (Estilux, Fa. Kulzer): Als Füllstoff dient ein silanisiertes Lithiumaluminiumglas einer mittleren Korngröße von ca. 4 µm. Der Füllstoffgehalt liegt bei ca. 75 % (m/m).

Hybrid-Composite (Degufill H, Degussa):
Als Füllstoff dienen silanisiertes Bariumaluminiumsilikatglas einer mittleren Korngröße von ca. 2 µm, welches aber zu 100 % feiner als 5 µm ist, sowie silanisiertes, hochdisperses $SiO_2$. Der Füllgrad an Glas beträgt ca. 70 % (m/m), der an hochdispersem $SiO_2$ ca. 11 % (m/m). Daraus ergibt sich ein Gesamtgehalt an anorganischen Füllstoffen von ca. 80 % (m/m).

Microfüller Composite (Durafill, Fa. Kulzer):
Als Füllstoff dient silanisiertes, hochdisperses $SiO_2$ einer mittleren Korngröße zwischen 0,01 bis 0,04 µm. Der Füllgrad beträgt ca. 50 % (m/m).

Die Aushärtung aller Massen erfolgte mit dem Lichtgerät Degulux Degussa und einer Bestrahlungszeit von 40 Sekunden.

Heißhärtende Laborversuchsprodukte = VP (Angaben in Gewichtsteilen):

VP1: 17 Bis-GMA
7,7 TEDMA
75 Bariumaluminiumsilikatglas, silanisiert (mittlere Korngröße ca. 4 µm)
0,3 Dibenzoylperoxid

VP2: 35 Bis-GMA
14,7 TEDMA
50 hochdisperses $SiO_2$, silanisiert (mittlere Korngröße 0,01 - 0,04 µm)
0,3 Dibenzoylperoxid

Die Aushärtung dieser Pasten erfolgt analog zur Aushärtung der heißhärtenden erfindungsgemäßen Massen.

Prüfung und Beurteilung der Polierbarkeit:
Von allen Materialien wurden Prüfkörper eines Durchmessers von 15 mm und einer Dicke von 3 mm hergestellt. Die Oberflächen aller Prüfkörper wurden zunächst mit feinem Schleifpapier (600 grit) gleichmäßig beschliffen. Dann wurden sie unter Wasser mit feinstteiligem Aluminiumoxid (mittlere Teilchengröße 0,04 µm) auf einem Baumwolltuch poliert.

Die Polierbarkeit wurde visuell beurteilt und mittels einer Punkteskala zwischen 1 bis 5 benotet, wobei 1 = matt und 5 = hochglänzend ist.

TABELLE I

Eigenschaften der erfindungsgemäßen Dentalmassen im Vergleich zu Handelsprodukten sowie zu konventionellen und microgefüllten heißhärtenden Dentalmassen

| Beispiel | 15 | 16 | 17 | 18 | 19 | 20 | 21 | 22 | Estilux | Dura-fill | Degu-fill H | VP1 | VP2 |
|---|---|---|---|---|---|---|---|---|---|---|---|---|---|
| Biegefestigkeit [N/mm²] DIN 13 | 140 | 135 | 158 | 155 | 120 | 110 | 115 | 112 | 100 | 62 | 110 | 140 | 65 |
| Biegemodul [N/mm²] DIN | 7900 | 6800 | 7300 | 7500 | 6200 | 5800 | 5200 | 5400 | 9700 | 3200 | 8000 | 11000 | 3900 |
| Druckfestigkeit [N/mm²] | 450 | 480 | 475 | 480 | 390 | 400 | 410 | 430 | 300 | 350 | 320 | 350 | 410 |
| Vickers-Härte HV5 [N/mm²] DIN | 810 | 850 | 810 | 760 | 580 | 550 | 520 | 490 | 660 | 290 | 680 | 660 | 330 |
| Polierbarkeit | 5 | 5 | 5 | 5 | 5 | 5 | 5 | 5 | 1 | 4 | 3 | 1 | 4 |
| Transparenz % | | | | | | | | | | | | | |
| bei 460 nm | 12,0 | 11,4 | 25,5 | 27,9 | 10,3 | 10,5 | 28,4 | 29,5 | x | x | 9,5 | - | - |
| bei 770 nm | 34,5 | 33,7 | 53,2 | 55,2 | 30,2 | 30,0 | 55,8 | 57,3 | x | x | 23,0 | - | - |
| (gemessen nach CIELAB) | | | | | | | | | | | | | |

x) nicht bestimmbar, da eingefärbt
-) nicht ermittelt

## Patentansprüche

1. Pastöses, in Gegenwart eines Initiators zu einer auf Hochglanz polierbaren Masse aushärtbares Dentalmaterial aus einem polymerisierbaren organischen Bindemittel und einem Al-haltigen Organopolysiloxan als Füllstoff, der aus Einheiten der Formel

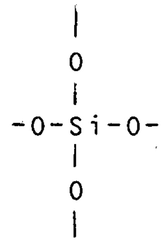

(I)

und Einheiten der Formel

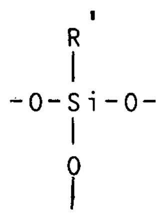

(II)

wobei R' für eine mit einem Acrylat- oder Methacrylatrest verbundene lineare oder verzweigte Alkylgruppe mit 1 bis 6 C-Atomen oder für einen einfach olefinisch ungesättigten, vorzugsweise endständig ungesättigten linearen, gegebenenfalls verzweigten Kohlenwasserstoffrest mit 2 bis 8 C-Atomen oder für einen zyklischen, einfach olefinisch ungesättigten Kohlenwasserstoffrest mit 5 - 8 C-Atomen oder für eine lineare, gegebenenfalls verzweigte Alkylgruppe mit 1 bis 8 C-Atomen, eine Cycloalkylengruppe mit 5 bis 8 C-Atomen, eine Phenylgruppe oder eine Alkylarylgruppe steht und/oder Einheiten der Formel

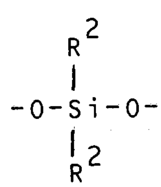

(III)

in denen $R^2$ eine Methyl-, Ethyl-, Propyl- oder Phenylgruppe repräsentiert und - bei jeder der Zusammensetzungen - Einheiten der Formel

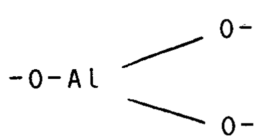

oder

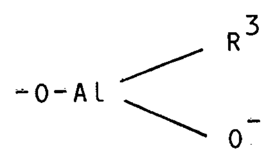

(IV)

in denen $R^3$ eine lineare oder verzweigte Alkylgruppe mit 1 bis 5 C-Atomen oder eine Phenylgruppe ist, vorliegen und die freien Valenzen der an die Silicium- und Aluminiumatome gebundenen Sauerstoffatome bei den Einheiten (I), (II) und/oder (III) sowie (IV) wie bei Heterosiloxangerüsten durch ein Siliciumatom einer gleichen oder einer unterschiedlichen Einheit oder durch ein Aluminiumatom abgesättigt werden, wobei das Verhältnis der Siliciumatome aus den Einheiten der Formel (I) zu der Summe der Siliciumatome der Einheiten (II) und (III) 3 : 1 bis 100 : 1 und das Verhältnis der Summe der Siliciumatome aus den Einheiten (I), (II) und (III) zu den Aluminiumatomen aus den Einheiten (IV) 2 : 1 bis 200 : 1 beträgt, besteht,
**dadurch gekennzeichnet**,
daß der darin enthaltene Füllstoff in Gestalt eines statistischen Coplykondensats erhältlich ist, indem man ein Alkoxysilan der allgemeinen Formel

$Si(OR^4)_4$ (V)

und ein Alkoxysilan der allgemeinen Formel

$R^1 - Si(OR^4)_3$ (VI)

in der R' dieselbe Bedeutung wie in Formel (II) hat, und/oder ein Alkoxysilan der allgemeinen Formel

$(R^2)_2Si(OR^4)_2$ (VII)

in der $R^2$ dieselbe Bedeutung wie in Formel (III) hat sowie eine Aluminiumverbindung der allgemeinen Formel

$Al(OR^4)_3$ bzw. $AlR^3(OR^4)_2$ (VIII)

in der $R^3$ dieselbe Bedeutung wie in Formel (IV) hat, wobei $R^4$ für eine lineare oder verzweigte Alkylgruppe mit 1 bis 5 C-Atomen steht, in einem weitgehend wassermischbaren, aber die Verbindungen nach Formel (V), (VI), (VII) sowie (VIII) lösenden Lösungsmittel auflöst, dann das Reaktionsgemisch in Gegenwart eines sauren, basischen oder Metall-haltigen Katalysators unter Rühren bei einer bestimmten Temperatur im Bereich von Raumtemperatur bis 200 °C vorkondensiert, durch Zusatz von gegebenenfalls Säure- oder Base-haltigem Wasser die Hydrolyse und vollständige Polykondensation durchführt und den gebildeten Feststoff, gegebenenfalls nach Zusatz weiteren Lösungsmittels oder Wassers, noch 1 Stunde bis zu 6 Stunden bei 60 °C bis 200 °C, bei Normaldruck oder einem Druck rührt, welcher der Summe der Partialdrucke bei der jeweiligen Temperatur entspricht, dann das gebildete Organopolysiloxan, gegebenenfalls nach einem Wechsel des Mediums und/oder pH-Wertes, noch 1 Stunde bis 5 Tage bei 60 °C bis 250 °C in flüssiger Phase nachbehandelt, dann nach gängigen Techniken von der flüssigen Phase abtrennt, gegebenenfalls wäscht, bei Raumtemperatur bis 200 °C, gegebenenfalls unter Schutzgasatmosphäre oder im Vakuum trocknet, gegebenenfalls anschließend 1 bis 100 Stunden bei Temperaturen von 150 °C bis 250 °C unter Schutzgasatmosphäre oder im Vakuum tempert, gegebenenfalls mahlt und/oder klassifiziert, wobei man das von der flüssigen Phase abgetrennte und gegebenenfalls gewaschene Organopolysiloxan vor oder nach einer der Stufen Trocknung, Temperung, Mahlung, Klassifizierung, in Wasser, einem Wasser/Alkohol-Gemisch oder in reinem Alkohol, in Gegenwart eines sauren oder eines basischen Katalysators, bevorzugt in Gegenwart von Ammoniak oder Alkali- oder Erdalkalioxiden bzw. -hydroxiden über einen Zeitraum von 1 Stunde bis zu 5 Tagen bei Temperaturen von 60 °C bis 250 °C unter einem Druck, welcher der Summe der Partialdrucke bei der jeweiligen Temperatur entspricht, behandelt und vor der weiteren Aufarbeitung säure- bzw. laugefrei wäscht.

**2.** Dentalmaterial gemäß Anspruch 1,
**dadurch gekennzeichnet**,
daß sein Füllstoff erhältlich ist, indem Hydrolyse und Kondensation in Methanol, Ethanol, n- und i-Propanol, n- und i-Butanol und/oder n-Pentanol durchgeführt werden.

**3.** Dentalmaterial nach Anspruch 1 oder 2,
**dadurch gekennzeichnet**,
daß sein Füllstoff erhältlich ist, indem man die monomeren Komponenten nach Formel (V), (VI) und/oder (VII) sowie (VIII) ohne oder unter Verwendung eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer weitgehend wasserfreien Säure oder Base über einen Zeitraum von 5 Minuten bis zu 5 Tagen bei Raumtemperatur bis 200 °C vorkondensiert.

**4.** Dentalmaterial gemäß den Ansprüchen 1 bis 3,
**dadurch gekennzeichnet**,
daß sein Füllstoff in Gestalt eines Block-Copolykondensats erhältlich ist, indem man alle monomeren Komponenten nach Formel (V), (VI) und/oder (VII) sowie (VIII) jeweils unabhängig voneinander, oder in einer 2er - aber höchstens 3er-Kombination, ohne oder unter Verwendung eines die Ausgangsstoffe lösenden Lösungsmittels, bevorzugt eines zu den Alkoxygruppen korrespondierenden, linearen oder verzweigten Alkohols mit 1 bis 5 C-Atomen, in Gegenwart einer wasserfreien Säure oder Base über

einen Zeitraum von 5 Minuten bis zu 5 Tagen bei Raumtemperatur bis 200 °C vorkondensiert, die erhaltenen Kondensate vereinigt, dann gemeinsam über einen Zeitraum von 5 Min. bis zu 2 Tagen bei Raumtemperatur bis 200 °C nochmals vorkondensiert und hierauf nach Zusatz von gegebenenfalls Säure- oder Base-haltigen weiteren Lösungsmittels die Hydrolyse und vollständige Polykondensation gemäß Anspruch 1 durchführt.

**5.** Dentalmaterial gemäß den Ansprüchen 1 bis 4,
**dadurch gekennzeichnet**,
daß sein Füllstoff in Gestalt eines gemischten Copolykondensats erhältlich ist, indem man von den monomeren Komponenten nach Formel (V), (VI) und/oder (VII) sowie (VIII) mindestens ein Monomer aber höchstens 3 Monomere, voneinander unabhängig oder in Kombination miteinander, ohne oder unter Verwendung eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer weitgehend wasserfreien Säure oder Base über einen Zeitraum von 5 Minuten bis zu 5 Tagen bei Raumtemperatur bis 200 °C vorkondensiert, das erhaltene Vorkondensat bzw. die erhaltenen Vorkondensate und mindestens eine nicht vorkondensierte Komponente miteinander vereinigt, die vereinigten Komponenten dann über einen Zeitraum von 5 Minuten bis zu 2 Tagen bei Raumtemperatur bis 200 °C nochmals vorkondensiert und dann nach Zusatz von gegebenenfalls Säure- oder Base-haltigen Wassers und gegebenenfalls weiteren Lösungsmittels die Hydrolyse und vollständige Polykondensation gemäß Anspruch 1 durchführt.

**6.** Dentalmaterial gemäß den Ansprüchen 1 bis 5,
**dadurch gekennzeichnet**,
daß der Füllstoff in bezug auf die Einheiten (I) bis (IV) als statistisches Copolykondensat, Block-Copolykondensat oder als Gemisch aus diesen Formen vorliegt.

**7.** Dentalmaterial gemäß den Ansprüchen 1 bis 6,
**dadurch gekennzeichnet**,
daß $R^1$ in Formel (II) für die Gruppe

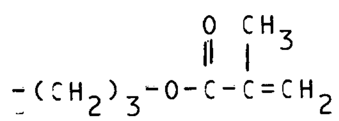

steht.

**8.** Dentalmaterial gemäß den Ansprüchen 1 bis 7,
**dadurch gekennzeichnet**,
daß als Füllstoff ein Organopolysiloxan verwendet wird, das aus Einheiten der Formel (I) und Einheiten der Formel (II) mit der Zusammensetzung

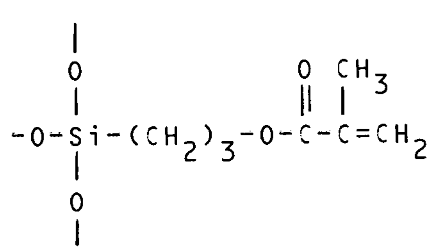

sowie Einheiten der Formel (IV) besteht, wobei das molare Verhältnis der Einheiten nach Formel (I) zu den Einheiten der Formel (II) 3 : 1 bis 100 : 1 und das Verhältnis der Summe der Siliciumatome aus den Einheiten (I) und (II) zu den Aluminiumatomen aus Einheiten (IV) 2 : 1 bis 200 : 1 beträgt.

**9.** Dentalmaterial gemäß Anspruch 1 oder 2,
**dadurch gekennzeichnet**,

daß als Füllstoff ein Organopolysiloxan verwendet wird, das aus Einheiten der Formel (I) und Einheiten der Formel (III) mit der Zusammensetzung

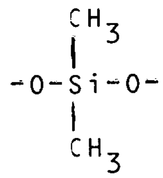

sowie Einheiten der Formel (IV) besteht, wobei das molare Verhältnis der Einheiten nach Formel (I) zu den Einheiten der Formel (III) 3 : 1 bis 100 : 1 und das Verhältnis der Summe der Siliciumatome aus den Einheiten (I) und (III) zu den Aluminiumatomen aus Einheiten (IV) 2 : 1 bis 200 : 1 beträgt.

**10.** Dentalmaterial gemäß den Ansprüche 1 bis 8,
**dadurch gekennzeichnet**,
daß der Füllstoff eine spezifische Oberfläche von 10 bis 250 $m^2/g$, vorzugsweise 30 bis 200 $m^2/g$, und eine Partikelgröße von 0,01 µm bis 100 µm, vorzugsweise 0,1 um bis 30 µm, aufweist.

**11.** Verwendung des Dentalmaterials nach vorstehenden Ansprüchen zur Fertigung von Zahnfüllungen, Inlays, Verblendungen, Zahnversiegelungen, Überzügen zum Schutz von Zahnoberflächen, Kronen, Brücken, Zahnprothesen, künstlichen Zähnen, Klebern zur Befestigung von Inlays, Kronen und Brücken sowie zum Aufbau von Zahnstümpfen.

## Claims

**1.** Paste-form dental material which in the presence of an initiator is curable to a mass polishable to a high gloss, comprising a polymerisable organic binder and an Al-containing organopolysiloxane as the filler which comprises units of the formula

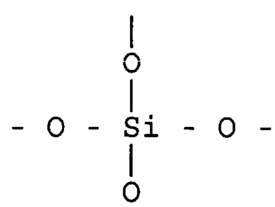

(I)

and units of the formula

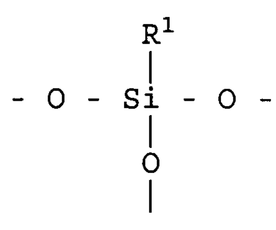

(II)

wherein $R^1$ stands for a linear or branched alkyl group bonded with an acrylate or methacrylate radical and having 1 to 6 C atoms or for a linear, optionally branched, unsaturated hydrocarbon radical having 2 to 8 C atoms and having a single olefinic bond, preferably in the end position, or for a cyclic unsaturated hydrocarbon radical having 5 to 8 C atoms and having a single olefinic bond, or for a linear, optionally branched alkyl group having 1 to 8 C atoms, a cycloalkylene group having 5 to 8 C atoms, a phenyl group or an alkylaryl group, and/or units of the formula

$$\begin{array}{c} R^2 \\ | \\ -\ O\ -\ Si\ -\ O\ - \\ | \\ R^2 \end{array} \qquad (III)$$

in which $R^2$ represents a methyl, ethyl, propyl or phenyl group and there are present - in each of the compositions - units of the formula

$$-\ O\ -\ Al \begin{array}{l} /\ \ O\ - \\ \\ \backslash\ \ O\ - \end{array} \quad \text{or} \quad -\ O\ -\ Al \begin{array}{l} /\ \ R^3 \\ \\ \backslash\ \ O\ - \end{array} \qquad (IV)$$

in which $R^3$ is a linear or branched alkyl group having 1 to 5 C atoms or a phenyl group, and the free valences of the oxygen atoms bonded to the silicon atoms and aluminium atoms in the case of the units (I), (II) and/or (III) and (IV) are, as in the case of heterosiloxane skeletons, satisfied by a silicon atom of a unit which is the same or different or by an aluminium atom, wherein the ratio of the silicon atoms from the units of the formula (I) to the sum of the silicon atoms of the units (II) and (III) is from 3 : 1 to 100 : 1, and the ratio of the sum of the silicon atoms from the units (I), (II) and (III) to the aluminium atoms from the units (IV) is from 2 : 1 to 200 : 1, characterised in that the filler contained therein is obtainable in the form of a random copolycondensate in that an alkoxysilane of the general formula

$Si(OR^4)_4$ (V)

and an alkoxysilane of the general formula

$R^1 - Si(OR^4)_3$ (VI)

in which $R^1$ denotes the same as in formula (II), and/or an alkoxysilane of the general formula

$(R^2)_2Si(OR^4)_2$ (VII)

in which $R^2$ denotes the same as in formula (III) and an aluminium compound of the general formula

$Al(OR^4)_3$ or $AlR^3(OR^4)_2$ (VIII)

in which $R^3$ denotes the same as in formula (IV), wherein $R^4$ stands for a linear or branched alkyl group having 1 to 5 C atoms, are dissolved in a solvent which is largely water-miscible but dissolves the compounds according to the formulae (V), (VI), (VII) and (VIII), the reaction mixture is then precondensed in the presence of an acid, basic or metal-containing catalyst, with stirring, at a specified temperature within the range from room temperature to 200 °C, hydrolysis and complete polycondensation are performed by the addition of optionally acid- or base-containing water, and the solid formed is stirred, optionally after the addition of further solvent or water, for a further 1 to 6 hours at from 60 to 200 °C, at standard pressure or at a pressure which corresponds to the sum of the partial pressures at the respective temperature, the organopolysiloxane formed is then post-treated, optionally after changing the medium and/or pH, for a further 1 hour to 5 days at from 60 to 250 °C in the liquid phase, and is then separated from the liquid phase using current techniques, is optionally washed, is dried at from room temperature to 200 °C, optionally in a protective gas atmosphere or under vacuum, is then optionally tempered for from 1 to 100 hours at temperatures of from 150 to 250 °C in a protective gas atmosphere or under vacuum, is optionally ground and/or classified, wherein, before or after one of the steps comprising drying, tempering, grinding, classifying, the organopolysiloxane separated from the liquid phase and optionally washed is treated in water, a water/alcohol mixture or pure alcohol, in the

presence of an acid or basic catalyst, preferably in the presence of ammonia or alkali metal or alkaline-earth metal oxides or hydroxides for a period of from 1 hour to 5 days at temperatures of from 60 to 250 °C at a pressure which corresponds to the sum of the partial pressures at the respective temperature, and, before further working-up, is washed until no acid or alkali remains.

2. Dental material according to Claim 1, characterised in that the filler thereof is obtainable in that hydrolysis and condensation are performed in methanol, ethanol, n- and i-propanol, n- and i-butanol and/or n-pentanol.

3. Dental material according to Claim 1 or 2, characterised in that the filler thereof is obtainable in that the monomer components according to the formulae (V), (VI) and/or (VII) and (VIII) are precondensed with or without the use of a solvent which dissolves the starting substances, preferably a linear or branched alcohol corresponding to the alkoxy groups and having 1 to 5 C atoms, in the presence of a largely anhydrous acid or base for a period of from 5 minutes to 5 days at from room temperature to 200 °C.

4. Dental material according to Claims 1 to 3, characterised in that the filler thereof is obtainable in the form of a block copolycondensate in that all the monomer components according to the formulae (V), (VI) and/or (VII) and (VIII), in each case independently of one another, or in a combination of 2 - but at the most 3 -, are precondensed in the presence of an anhydrous acid or base for a period of from 5 minutes to 5 days at from room temperature to 200 °C, with or without the use of a solvent which dissolves the starting substances, preferably a linear or branched alcohol corresponding to the alkoxy groups and having 1 to 5 C atoms, the condensates obtained are combined and are then precondensed again together for a period of from 5 minutes to 2 days at from room temperature to 200 °C, and the hydrolysis and complete polycondensation according to Claim 1 are performed following the addition of optionally acid- or base-containing further solvent.

5. Dental material according to Claims 1 to 4, characterised in that the filler thereof is obtainable in the form of a mixed copolycondensate in that a minimum of one but a maximum of 3 monomers of the monomer components according to the formulae (V), (VI) and/or (VII) and (VIII), independently of one another or in combination, are precondensed in the presence of a largely anhydrous acid or base for a period of from 5 minutes to 5 days at from room temperature to 200 °C, with or without the use of a solvent which dissolves the starting substances, preferably a linear or branched alcohol corresponding to the alkoxy groups and having 1 to 5 C atoms, the precondensate or precondensates obtained and at least one component which has not been precondensed are combined, the combined components are then precondensed again for a period of from 5 minutes to 2 days at from room temperature to 200 °C, and the hydrolysis and complete polycondensation according to Claim 1 are performed following the addition of optionally acid- or base-containing water and optionally further solvent.

6. Dental material according to Claims 1 to 5, characterised in that the filler is present in relation to the units (I) to (IV) as a random copolycondensate, a block copolycondensate or a mixture of the latter forms.

7. Dental material according to Claims 1 to 6, characterised in that $R^1$ in formula (II) stands for the group

$$-(CH_2)_3-O-\overset{\underset{\|}{O}}{C}-\overset{\underset{|}{CH_3}}{C}=CH_2.$$

8. Dental material according to Claims 1 to 7, characterised in that as the filler an organopolysiloxane is used which comprises units of the formula (I) and units of the formula (II) having the composition

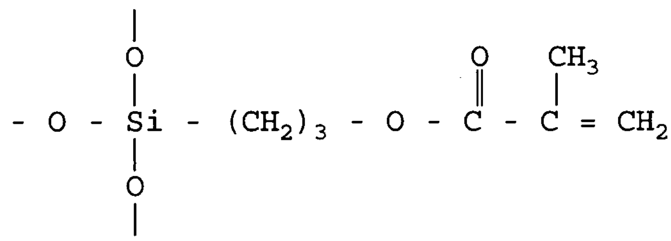

and units of the formula (IV), wherein the molar ratio of the units according to the formula (I) to the units of the formula (II) is from 3 : 1 to 100 : 1 and the ratio of the sum of the silicon atoms from the units (I) and (II) to the aluminium atoms from the units (IV) is from 2 : 1 to 200 : 1.

**9.** Dental material according to Claim 1 or 2, characterised in that as the filler an organopolysiloxane is used which comprises units of the formula (I) and units of the formula (III) having the composition

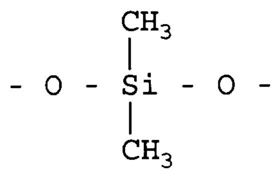

and units of the formula (IV), wherein the molar ratio of the units according to the formula (I) to the units of the formula (III) is from 3 : 1 to 100 : 1 and the ratio of the sum of the silicon atoms from the units (I) and (III) to the aluminium atoms from the units (IV) is from 2 : 1 to 200 : 1.

**10.** Dental material according to Claims 1 to 8, characterised in that the filler exhibits a specific surface of from 10 to 250 $m^2/g$, preferably from 30 to 200 $m^2/g$, and a particle size of from 0.01 $\mu m$ to 100 $\mu m$, preferably from 0.1 $\mu m$ to 30 $\mu m$.

**11.** Use of the dental material according to the preceding claims to prepare dental fillings, inlays, facings, dental seals, coatings to protect dental surfaces, crowns, bridges, dental prostheses, artificial teeth, adhesives for attaching inlays, crowns and bridges, and for building up dental stumps.

## Revendications

**1.** Matériau dentaire pâteux pouvant durcir en présence d'un excitant en une masse polissable à brillant spéculaire, constitué d'un liant organique polymérisable et d'un organopolysiloxane contenant de l'aluminium comme charge, qui se compose d'unités de formule :

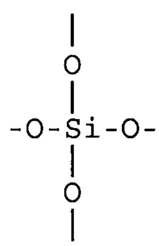

(I)

et d'unités de formule (II)

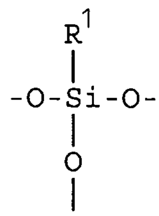 (II)

dans lesquelles $R^1$ représente un groupe alkyle linéaire ou ramifié, lié à un radical acrylate ou méthacrylate, ayant de 1 à 6 atomes de carbone ou un radical d'hydrocarbure non saturé une fois oléfiniquement, de préférence insaturé en position terminale, linéaire, éventuellement ramifié ayant de 2 à 8 atomes de C ou un radical d'hydrocarbure cyclique, non saturé une fois oléfiniquement ayant 5-8 atomes de C ou un groupe alkyle linéaire, éventuellement ramifié avec de 1 à 8 atomes de C, un groupe phényle, un groupe cycloalkylène avec de 5 à 8 atomes de C ou un groupe alkylaryle et/ou d'unités de formule (III).

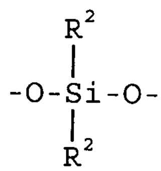 (III)

dans lesquelles $R^2$ représente un groupe méthyle, éthyle, propyle ou phényle, et pour chacune des compositions des unités de formule IV.

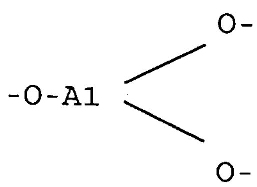 ou 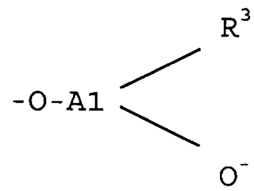 (IV)

dans lesquelles $R^3$ est un groupe alkyle linéaire ou ramifié avec de 1 à 5 atomes de C ou un groupe phényle, et les valences libres des atomes d'oxygène liés aux atomes de silicium et d'aluminium dans les unités (I), (II) et/ou (III) ainsi que (IV) sont saturés comme dans le cas des structures d'hétérosiloxane par un atomes de silicium d'une unité égale ou différente ou par un atome d'aluminium, le rapport des atomes de silicium des unités de formule (I) à la somme des atomes de silicium des unités (II) et (III) s'élevant de 3:1 à 100:1 et le rapport de la somme des atomes de silicium des unités (I), (II) et (III) aux atomes d'aluminium des unités (IV) de 2:1 à 200:1.
Caractérisé en ce que,

la charge contenue dans la structure d'un copolycondensat statistique est disponible en ce que l'on dissout un alcoxysilane de formule générale :

$Si(OR^4)_4$ (V)

et un alcoxysilane de la formule générale :

$R^1 - Si(OR^4)_3$ (VI)

dans laquelle $R^1$ a la même signification que dans la formule (II), et/ou un alcoxysilane de la formule générale :

$(R^2)_2Si(OR^4)_2$ (VII)

dans laquelle $R^2$ a la même signification que dans la formule (III), ainsi qu'un composé d'aluminium de la formule générale :

$Al(OR^4)_3$ ou $AlR^3(OR^4)_2$ (VIII)

dans laquelle $R^3$ a la même signification que dans la formule (IV), tandis que $R^4$ représente un groupe alkyle linéaire ou ramifié avec de 1 à 5 atomes de C,
dans un solvant largement miscible dans l'eau, mais dissolvant les composés selon les formules (V), (VI), (VII) ainsi que (VIII), qu'ensuite on précondense le mélange réactionnel en présence d'un catalyseur acide, basique ou contenant du métal en agitant , à une température déterminée dans la plage de la température ambiante jusqu'à 200 °C, qu'on réalise,par ajout d'eau contenant éventuellement de l'acide ou une base, l'hydrolyse et la polycondensation complète et qu'on agite la matière solide formée, éventuellement après addition de davantage de solvant ou d'eau, encore pendant 1 heure à 6 heures entre 60 °C et 200 °C, à pression normale ou à une pression, qui correspond à la somme des pressions partielles à la température respective, ensuite qu'on traite l'organopolysiloxane formé, éventuellement après un changement du milieu et/ou de la valeur du pH pendant encore de 1 heure à 5 jours entre 60 °C et 250 °C dans la phase liquide, puis qu'on le sépare de la phase liquide selon des techniques courantes, éventuellement qu'on le lave, qu'on le sèche entre la température ambiante et 200 °C éventuellement sous atmosphère protectrice ou sous vide, éventuellement ensuite qu'on le recuit pendant 1 à 100 heures à des températures de 150 °C à 250 °C sous atmosphère protectrice ou sous vide, éventuellement qu'on le broie et/ou qu'on le classifie, tandis qu'on traite l'organopolysiloxane séparé de la phase liquide et éventuellement lavé avant ou après une des étapes de séchage, recuit, broyage, classement, dans de l'eau, dans un mélange eau/alcool ou dans de l'alcool pur, en présence d'un catalyseur acide ou basique, de préférence en présence d'ammoniac ou d'oxyde ou d'hydroxydes de métal alcalin ou alcalino-terreux, pendant une durée de 1 heure à 5 jours à des températures de 60 °C à 250 °C à une pression, qui correspond à la somme des pressions partielles à la température considérée, et avant le retraitement suivant on lave sans acide ni lessive.

**2.** Matériau dentaire selon la revendication 1, caractérisé en ce que,
sa charge peut être obtenue en réalisant l'hydrolyse et la condensation dans du méthanol, de l'éthanol, du n- et i-propanol, du n- et i-butanol et/ou du n-pentanol.

**3.** Matériau dentaire selon la revendication 1 ou 2, caractérisé en ce que,
sa charge peut être obtenue, en précondensant les composants monomères selon les formules (V), (VI) et/ou (VII) ainsi que (VIII) sans ou avec utilisation d'un solvant dissolvant les matières de départ, de préférence un alcool linéaire ou ramifié correspondant aux groupes alcoxy ayant de 1 à 5 atomes de C, en présence d'un acide largement acide ou base pendant un intervalle de temps de 5 minutes à 5 jours à température ambiante jusqu'à 200 °C.

**4.** Matériau dentaire selon les revendications 1 à 3, caractérisé en ce que,
sa charge dans la structure d'un copolycondensat en bloc peut être obtenue, en précondensant tous les composants monomères selon les formules obtenues, en précondensant tous les composants monomères selon les formules (V), (VI) et/ou (VII) ainsi que (VIII) respectivement indépendamment les uns des autres ou dans une combinaison à deux ou au maximum à trois, sans ou avec utilisation d'un solvant dissolvant les matières de départ, de préférence un alcool linéaire ou ramifié correspondant aux groupes alcoxy, ayant de 1 à 5 atomes de C, en présence d'un acide anhydre ou une base pendant une durée de 5 minutes jusqu'à 5 jours à température ambiante jusqu'à 200 °C, en réunissant les condensats obtenus, en précondensant ensuite encore une fois en commun pendant une durée de 5 minutes jusqu'à 2 jours à température ambiante jusqu'à 200 °C et ensuite après addition éventuellement d'autre solvant contenant de l'acide ou une base en effectuant l'hydrolyse et la polycondensation selon la revendication 1.

**5.** Matériau dentaire selon les revendications 1 à 4, caractérisé en ce que,
sa charge dans la structure d'un copolycondensat mixte peut être obtenue, en précondensant parmi les composants monomères selon les formules (V), (VI) et/ou (VII) ainsi que (VIII) au moins un monomère mais au maximum trois monomères, indépendamment les uns des autres, sans ou avec utilisation d'un solvant dissolvant les matières de départ, de préférence un alcool linéaire ou ramifié, correspondant aux groupes alcoxy, ayant de 1 à 5 atomes de C, en présence d'un acide largement anhydre ou d'une

base pendant une durée de 5 minutes à 5 jours à la température ambiante jusqu'à 200°C, en réunissant le précondensat obtenu ou les précondensats obtenus et au moins un composant non précondensé, en précondensant ensuite encore une fois les composants réunis pendant une période de 5 minutes à 2 jours à la température ambiante jusqu'à 200°C et puis après addition éventuellement d'eau contenant de l'acide ou une base et éventuellement encore du solvant en réalisant l'hydrolyse et la polycondensation complète selon la revendication 1.

**6.** Matériau dentaire selon les revendications 1 à 5, caractérisé en ce que,
la charge se présente en ce qui concerne les unités (I) à (IV) sous forme de copolycondensat statistique, copolycondensat en bloc ou de mélange de ces formes.

**7.** Matériau dentaire selon les revendications 1 à 7, caractérisé en ce que,
$R^1$ dans la formule (II) correspond au groupe

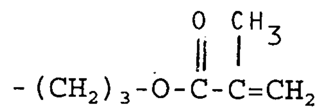

**8.** Matériau dentaire selon les revendications 1 à 7, caractérisé en ce que,
comme charge on utilise un organopolysiloxane, qui est composé d'unités de la formule (I) et d'unités de la formule (II) avec la composition

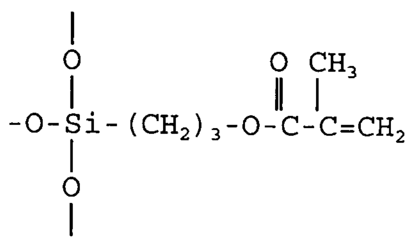

ainsi que d'unités de la formule (IV), tandis que le rapport molaire des unités selon la formule (I) aux unités de la formule (II) se monte à 3:1 jusqu'à 100:1 et le rapport de la somme des atomes de silicium des unités (I) et (II) aux atomes d'aluminium des unités (IV) est 2:1 à 200:1.

**9.** Matériau dentaire selon les revendications 1 ou 2, caractérisé en ce que,
comme charge on utilise un organopolysiloxane, qui se compose d'unités de la formule (I) et d'unités de la formule (III) avec la composition

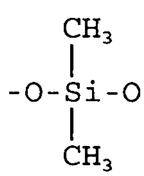

ainsi que d'unités de la formule (IV), tandis que le rapport molaire des unités selon la formule (I) aux unités de la formule (III) s'élève à 3:1 jusqu'à 100:1 et le rapport de la somme des atomes de silicium des unités (I) et (III) aux atomes d'aluminium des unités (IV) s'élève de 2:1 à 200:1.

**10.** Matériau dentaire selon les revendications 1 à 8, caractérisé en ce que la charge à une surface spécifique de 10 à 250 $m^2/g$, de préférence de 30 à 200 $m^2/g$, et une taille de particule de 0,01 µm à 100 µm, de préférence de 0,1 µm à 30 µm.

**11.** Utilisation du matériau dentaire selon les revendications précédentes, pour la réalisation de remplissage dentaires, d'inlays, de parements, de colmatages dentaires, de revêtements pour la protection des surfaces dentaires, de couronnes, de bridges, de prothèses dentaires, de dents artificielles, de colles pour la fixation d'inlays, couronnes et bridges ainsi que pour la construction de chicots dentaires.